# EUROPEAN PATENT APPLICATION

(11) **EP 2 415 454 A1**
(43) Date of publication of application: **08.02.2012**
(21) Application number: 10008166.0
(22) Date of filing: 05.08.2010
(51) Int. Cl.: A61K 8/60, A61K 8/73, A61Q 5/12

(54) **Cleansing composition**

(71) Applicant: KPSS-Kao Professional Salon Services GmbH, 64297 Darmstadt (DE)
(72) Inventor: Tietjen, Ilka, 68549 Ilvesheim (DE); Molenda, Michael, 60318 Frankfurt (DE)
(74) Representative: Grit, Mustafa

(57) **Abstract**

The present invention is related to an aqueous cleansing composition for keratin fibres, especially human hair, comprising starch derived cationic polymer and at least once sorbitan surfactant. Accordingly, present invention is on an aqueous cleansing composition for keratin fibres especially for human hair comprising starch derived cationic polymer, preferably an oxidized starch derived cationic polymer and at least one sorbitan surfactant.

## Description

The present invention is related to an aqueous cleansing composition for keratin fibres, especially human hair, comprising starch derived cationic polymer and at least one sorbitan surfactant.

Cleansing compositions have been subject matter of quite many patent application. The problem addressed are conditioning and cleansing benefits. Although plurality of the solutions suggested for improved cleansing and conditioning there is still need for further improvement, especially in terms of foaming power, in particular foam creaminess, and conditioning effect on hair.

It is known in the prior art that cationic surfactants or polymers or combination of them are used as conditioning ingredients in cleansing Compositions in addition to the surfactants in the formula which brings about additional material costs and more importantly additional production costs because of requirement of preliminary mixings and need for heat for preparation of the solutions, especially in case of cationic polymers, and therefore cooling. A cleansing composition which principally does not require any of such is a big advantage in terms of costs and it efficiency.

Aim of the present invention is to provide an aqueous cleansing composition having optimal benefits in terms of foam properties in terms of its volume and creaminess and conditions hair even in the absence of cationic conditioning ingredients in terms of combability, smoothness, elasticity, softness, volume and body, and provides hair excellent shine.

Present inventors have surprisingly found that an aqueous cleansing composition comprising starch derived cationic polymer and at least one sorbitan surfactant has excellent foam properties and provides excellent conditioning effect to human hair.

Accordingly, the first object of the present invention is an aqueous cleansing composition comprising starch derived cationic polymer and at least one sorbitan surfactant.

Second object of the present invention is the use of a composition comprising starch derived cationic polymer and a sorbitan surfactant for cleansing and conditioning hair.

Third objective of the present invention is the process for cleansing and conditioning hair wherein a composition comprising starch derived cationic polymer and a sorbitan surfactant is applied onto hair and after massaging for up to 3 min, preferably 2 min and more preferably 1 min and most preferably less than 60 seconds rinsed off from hair.

Compositions of the present invention comprises at least one starch derived cationic polymer. Preferred is a cationic oxidized starch polymer known with it CTFA/INCI name hydroxypropyl Oxidized Starch PG-Trimonium chloride available from Graefe Chemie GmbH under the trade name Amylomer,

Concentration of starch derived cationic polymer in the compositions is in the range of 0.01 to 5%, preferably 0.02 to 4%, more preferably 0.05 to 3% and most preferably 0.1 to 2.5% by weight calculated to total composition.

Composition of the present invention comprises at least one sorbitan surfactant. Suitable non-limiting examples are esters of sorbitan such as sorbitan caprylate, sorbitan cocoate, sorbitan diisostearate, sorbitan dioleate, sorbitan distearate, sorbitan isostearate, sorbitan laurate, sorbitan oleate, sorbitan olivate, sorbitan palmitate, sorbitan palmate, sorbitan sesquicaprylate, sorbitan sesquiisostearate, sorbitan sesquioleate, sorbitan sesquistearate and sorbitan stearate, as well as ethoxylated sorbitan surfactants such as PEG-20 sorbitan cocoate, PEG-40 sorbitan diisostearate, PEG-2 sorbitan isostearate, PEG-5 sorbitan isostearate, PEG-20 sorbitan isostearate, PEG-40 sorbitan laurate, PEG-10 sorbitan laurate, PEG-44 sorbitan laurate, PEG-75 sorbitan laurate, PEG-80 sorbitan laurate, PEG-3 sorbitan oleate, PEG-6 sorbitan oleate, PEG-20 sorbitan oleate, PEG-40 sorbitan oleate, PEG-80 sorbitan palmitate, PEG-3 sorbitan stearate, PEG-4 sorbitan stearate, PEG-6 sorbitan stearate, PEG-40 sorbitan stearate, and PEG-60 sorbitan stearate, and as well as Polysorbate 20, Polysorbate 21, Polysorbate 40, Polysorbate 60, Polysorbate 61, Polysorbate 65, Polysorbate 80, Polysorbate 81, and Polysorbate 85. Preferred are sorbitan caprylate, sorbitan cocoate, sorbitan diisostearate, sorbitan dioleate, sorbitan distearate, sorbitan isostearate, sorbitan laurate, sorbitan oleate, sorbitan olivate, sorbitan palmitate, sorbitan palmate, sorbitan sesquicaprylate, sorbitan sesquiisostearate, sorbitan sesquioleate, sorbitan sesquistearate, sorbitan stearate, PEG-20 sorbitan cocoate, PEG-40 sorbitan diisostearate, PEG-2 sorbitan isostearate, PEG-5 sorbitan isostearate, PEG-20 sorbitan isostearate, PEG-40 sorbitan laurate, PEG-10 sorbitan laurate, PEG-44 sorbitan laurate, PEG-75 sorbitan laurate, PEG-80 sorbitan laurate, PEG-3 sorbitan oleate, PEG-6 sorbitan oleate, PEG-20 sorbitan oleate, PEG-40 sorbitan oleate, PEG-80 sorbitan palmitate, PEG-3 sorbitan stearate, PEG-4 sorbitan stearate, PEG-6 sorbitan stearate, PEG-40 sorbitan stearate, and PEG-60 sorbitan stearate.

Preferably sorbitan surfactant is a sorbitan sesqui ester and more preferably it is sorbitan sesquicaprylate.

Concentration of at least one sorbitan surfactant is in the range of 0.1 to 15%, by weight, calculated to total composition. Preferably, the concentration of sorbitan surfactant is from 0.2 to 10% by weight, more preferably 0.25 to 7.5% by weight and most preferably 0.5 to 5% by weight, calculated to total composition. It should be noted that the compositions of the present invention can comprise more than one sorbitan surfactant and the above mentioned concentrations are valid for the total sorbitan surfactant concentration.

Cleansing compositions of the present invention comprise at least one anionic surfactant at a concentration range of 0.5 to 50%, preferably 1 to 40% and more preferably 1 to 30%, and most preferably 1 to 20% by weight, calculated to the total composition.

In principal any anionic surfactant is suitable within the meaning of the present invention. As mentioned above with the term anionic surfactant any anionic surfactants are meant other than amino acid surfactants. Nonlimiting examples are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂₋C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₇ - (C₂H₄O)ₙ- O - CH₂COOX,

wherein R₇ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 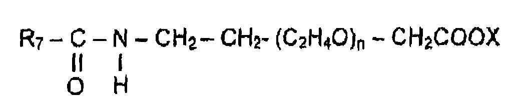 wherein R and X have the above meanings, and n is in particular a number from 1 to 10, preferably 2.5 to 5.

Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.

Further suitable anionic surfactants are amino acid surfactants of the following structure 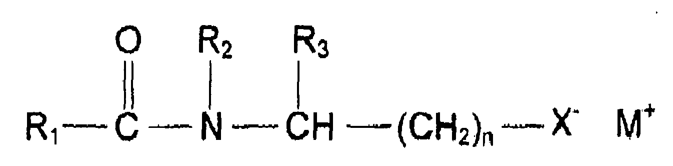 wherein R₁ is a saturated or unsaturated, straight or branched alkyl chain with 7 to 17 C atoms, R₂ is H or a methyl, R₃ is H, COO⁻ M⁺, CH₂COO⁻ M or COOH, n is 0 to 2, X is COO⁻ or SO₃⁻ and M is independent from each other H, sodium or potassium,

Suitably amino acid surfactant types are taurate, glutamate, alanin or alaninate, sarcosinate, aspartate surfactants, and mixtures thereof. Preferred are taurate, glutamate and sarcosinate surfactants and mixtures thereof. More preferred are taurates and glutamates and most preferred is glutamate type surfactants,

Suitable taurate surfactants are potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, and sodium methyl stearoyl taurate and mixtures thereof.

Suitable glutamate surfactants are dipotassium capryloyl glutamate, dipotassium undecylenoyl glutamate, disodium capryloyl glutamate, disodium cocoyl glutamate, disodium lauroyl glutamate, disodium stearoyl glutamate, disodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, and sodium undecylenoyl glutamate and mixtures thereof.

Suitable alanine or alaninate surfactants are cocoyl methyl β-alanine, lauroyl β-alanine, lauroyl methyl β-atanine, myristoyl β-alanine, potassium lauroyl methyl β-alanine, sodium cocoyl alaninate, sodium cocoyl methyl β-alanine and sodium myristoyl methyl β- alanine and mixtures thereof,

Suitable glycine surfactants are palmitoyl glycine, sodium lauroyl glycine, sodium cocoyl glycine, sodium myristoyl glycine, potassium lauroyl glycine, and potassium cocoyl glycine and mixtures thereof.

Suitable sarcosinate surfactants are potassium lauroyl sarcosinate, potassium cocoyl sarcosinate, sodium cocoyl sarcosinate, sodium lauroyl sarcosinate, sodium myristoyl sarcosinate, and sodium palmitoyl sarcosinate and mixtures thereof.

Suitable aspartate surfactants are sodium lauroyl aspartate, sodium myristoyl aspartate, sodium cocoyl aspartate, sodium caproyl aspartate disodium lauroyl aspartate, disodium myristoyl aspartate, disodium cocoyl aspartate, disodium caproyl aspartate, potassium lauroyl aspartate, potassium myristoyl aspartate, potassium cocoyl aspartate, potassium caproyl aspartate, dipotassium lauroyl aspartate, dipotassium myristoyl aspartate, dipotassium cocoyl aspartate, and dipotassium caproyl aspartate and mixtures thereof.

It should be noted that compositions of the present invention can also comprise mixture of several type of amino acid surfactants such as mixture of glutamate, and taurate surfactants, or mixture of taurate, glutamate and sarcosinate surfactants etc,

The most preferred anionic surfactants within the meaning of the present invention are those of alkyl ether sulphates such as lauryl ether sulphate sodium salt, and aminoacid surfactants.

It is also possible to use mixtures of several anionic surfactants, for example an ether sulfate and a polyether carboxylic acid or alkyl amidoether carboxylic acid or amino acid surfactant in an mixture.

It has further been observed that optimum foam and hair conditioning effects are related to the weight ratio of the anionic surfactant to sorbitan surfactant. Therefore, in the preferred embodiment of the present invention, weight ratio of anionic surfactant to sorbitan surfactant is preferably in the range of 10:1 to 1:10 and more preferably in the range of 8:1 to 1:8 and most preferably in the range of 5:1 to 1:5.

In a preferred embodiment of the present invention, cleansing composition of the present invention comprises at least one anionic surfactant as mentioned above and at least one nonionic surfactant in addition to the sorbitan surfactant. Nonionic surfactants other than sorbitan surfactants are suitable at a concentration of 1 to 15%, in particular from 1 to 10% by weight, calculated to the total composition.

Nonionic surfactants especially suited in the cleansing compositions according to the invention are alkyl polyglucosides of the general formula

R₈-O-(R₉O)ₙ-Zₓ,

wherein R₈ is an alkyl group with 8 to 18 carbon atoms, R₉ is an ethylene or propylene group, Z is a saccharide group with 5 to 6 carbon atoms, n is a number from 0 to 10 and x is a number between 1 and 5.

These alkyl polyglucosides are known in particular as excellent skin-campatible, foam improving agents in liquid detergents and body cleansing compositions. Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactants are, suitable for the cleansing compositions of the present invention, long-chain fatty acid dialkanolamides, such as coco fatty acid diethanolamide and myristic fatty acid diethanolamide.

Further suitable nonionic surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, pr also amineoxides with ethyleneoxide and/or propyleneoxide groups in the alkyl chain. Such amineoxides are on the market, for example, under the trade names "Ammonyx^{®}", "Aromox^{®}" or "Genaminox^{®}.

Further nonionic surfactants useful in the compositions according to invention are C₁₀-C₂₂-fatty alcohol ethoxylate. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2,5 and about 25, preferably about 10 and about 20. -

The most preferred non-ionic surfactants are alkyl polyglucosides such as decyl, cocoyl polyglucoside and ethoxylated fatty alcohols such as laureth-16.

In a further preferred embodiment of the present invention, cleansing composition of the present invention comprises at least one anionic, at least one nonionic surfactant and at least one amphoteric or zwitterionic surfactant.

Amphoteric or zwitterionic surfactants, are present at a concentration of 0.5 % to about 15 %, preferably 1 % to about 10 %, by weight, calculated to the total composition. It has especially been found out that addition of zwitterionic or amphoteric surfactants enhances foam feeling in terms of creaminess, foam volume and as well as skin compatibility are also improved. For achieving milder formulations anionic surfactant, especially of sulphate types, to amphoteric surfactant ratio should be in the range of 10:1 1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids; such as cocoaminoacetate, cocoaminopropionate and sodium cocoamphopropionate and -acetate have also proven suitable.

In detail, suitable betaine surfactants are of general structure 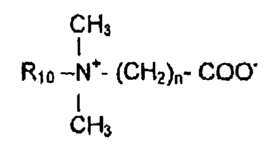 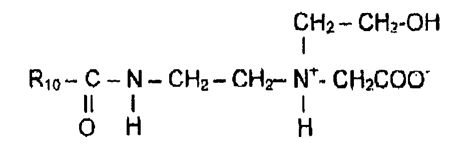 wherein R₁₀ is a C₈-C₁₈-alkyl group and n is 1 to 3;
sulfobetaines of the structure 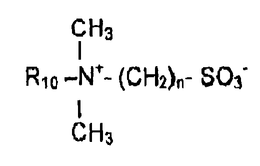 wherein R₁₀ and n are same as above;
and amidoalkyl betaines of the structure 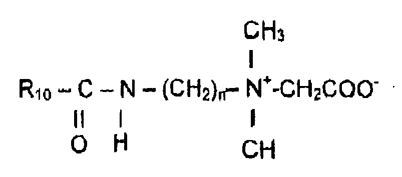 wherein R₁₀ and n are same as above.

The most preferred amphoteric surfactants are alkyl betaines such as lauryl betaine and alkyl amido betaines such as cocamidopropyl betaine.

In a further preferred form of the present invention, cleansing composition comprises at least one anionic surfactant especially of alkyl ether sulphate type, at least one amphoteric surfactant especially alkyl amido alkyl betaine type and at least one non-ionic surfactant especially an alkyl polyglucoside type in the above mentioned concentration ranges, certainly in addition to the amino acid surfactant and sorbitan surfactant.

Although it is not required for the performance, cleansing composition of the present invention may comprise hair-conditioning agents. Conditioning agents can be selected from oily substances, non-ionic substances, cationic amphiphilic ingredients, cationic polymers other than the one described above or their mixtures.

Oily substances are selected from such as silicone oils, either volatile or non-volatile, natural and synthetic oils. Among silicone oils those can be added to the compositions include dimethicone, dimethiconol, polydimethylsiloxane, DC fluid ranges from Dow Corning, arylated silicones such as phenyl trimethicone or any other silicone with up to 5 aryl, preferably phenyl, group in its molecule such as trimethyl pentaphenyl trisiloxane, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents can be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁₁ CO (O CH₂ CH₂)ₙ OH

or

R₁₁ CO (O CH₂ CH₂)ₙ O OC R₁₂

where R₁₁ and R₁₂ are independent from each other saturated, unsaturated or branched or non-branched alkyl chain with 7 to 21 C atoms and n is typically 2-100.

In one of the preferred form of the present invention, cleansing compositions comprise at least one additional cationic polymer as a conditioning agent. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15. Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquatemium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum an its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11010, 30 44 738 and 32 17 059, as well as to the products describes in EP-A 337 354 on pages 3 to 7. It is also possible to use mixtures of various cationic polymers.
The most preferred cationic polymers are those of cationic cellulose derivatives, cationic guar gum derivatives, cationic Caesalpinia spinosa gum derivatives, polyquaternium 6, polyquaternium 7, polyquaternium 67 and polyquaternium 70.

The cationic polymers also include the quaternized products of graft polymers from organopolysiloxanes and polyethyl oxazolines described in EP-A 524 612 and EP-A 640 643.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, silicone oil and derivatives and cationic surfactants is in the range of 0.01 to 5% by weight, preferably 0.01 to 3.5% by weight, more preferably 0.05 to 2.5% and most preferably 0.1 to 1.5% by weight calculated to the total composition. Most preferred conditioning agents are cationic polymers.

Aqueous cleansing composition may comprise at least one organic solvent such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, polypropyleneglycols, ethyleneglycol monoethylether, ethylene glycol monobutyl ether, ethylene glycol monophenyl ether, 1 -phenylethylalcohol, 2-phenylethylalcohol, o-methoxyphenol. The most preferred ones are benzyl alcohol and polypropylene glycols. Total concentration of organic solvents in the shampoo composition should not exceed 5% by weight, preferably in the range of 0.1 to 3%, more preferably 0.5 to 2,5% by weight calculated to total composition.

Aqueous cleansing composition of the present invention may comprise at least one glyceryl ether of the following formula 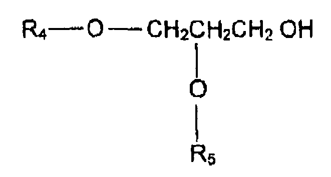 wherein R₄ is straight or branched, saturated or unsaturated alkyl chain with 4 to 24 C atoms, preferably 4 to 18 and more preferably 4 to 12 C atoms and R₅ is H, or straight or branched, saturated or unsaturated alkyl chain with 4 to 24 C atoms, 4 to 18 and more preferably 4 to 12 C atoms and most preferably R₅ is H, at a concentration of 0.1 to 15%, preferably 0.1 to 10% and more preferably 0.25 to 7.5% and most preferably 0.5 to 5% by weight calculated to total composition.

Suitable unlimited examples are glyceryl butyl ether, glyceryl isobutyl ether, glycerol tert-butyl ether, glyceryl pentyl ether, glyceryl isopentyl ether, glyceryl hexyl ether, glyceryl isohexyl ether, glyceryl heptyl ether, glyceryl octyl ether, glyceryl ethylhexyl ether, glyceryl nonyl ether, glyceryl decyl ether, glyceryl isodecyl ether, glyceryl lauryl ether, glyceryl myristyl ether, glyceryl palmityl ether, glyceryl stearyl ether and glyceryl behenyl ether and their mixtures. Most preferred are glyceryl butyl ether, glyceryl isobutyl ether, glyceryl tert-butyl ether, glyceryl pentyl ether, glyceryl isopentyl ether, glyceryl hexyl ether, glyceryl isohexyl ether, glyceryl heptyl ether, glyceryl octyl ether, glyceryl ethylhexyl ether, glyceryl nonyl ether, glyceryl decyl ether, glyceryl isodecyl ether are glyceryl lauryl ether, and their mixtures.

It should be noted that within the disclosure of the.present description, glyceryl decyl ether is used as synonym of decyl glycerine. For the other compounds in the above paragraph the same is valid.

Aqueous cleansing composition of the present invention may further comprise at least one fatty alcohol of the following formula

R6 - OH

wherein R₆ is straight or branched, saturated or unsaturated alkyl chain with 8 to 24, preferably 10 to 22, more preferably 12 to 18 and most preferably 12 to 16C atoms at a concentration of 0.1 to 5%, preferably 0.1 to 4% and more preferably 0.25 to 3% and most preferably 0.5 to 2.5% by weight calculated to total composition.

Suitable non-limiting preferred examples are decyl alcohol, myristyl alcohol, lauryl alcohol, cetyl alcohol, stearyl alcohol, behenyl alcohol, and arachidyl alcohol and their mixtures. More preferred are decyl alcohol, myristyl alcohol, lauryl alcohol, cetyl alcohol, and stearyl alcohol. Most preferred are decyl alcohol, myristyl alcohol and lauryl alcohol, and their mixtures.

Cleansing composition of the present invention may be pearly. Pearl-shiny appearance is achieved with those dispersed in cleansing conditioning compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kinds of mixtures are available commercially.

Solubilizers may be added to the compositions especially when oily substances are chosen as conditioning agents and fragrance oils with highly lipophilic properties. Typical solubilizers may be hydrogenated castor oil known with the trade mark Cremophor CO series from BASF. It should be noted that as well the surfactant mixture can be a good solubilizer for fragrance oils. Typical concentration of the solubilizers can be in the range of 0.01 - 2% by weight, preferably 0. 1 - 1 % by weight, calculated to total composition.

The cleansing composition may contain active ingredients selected from UV filters, moisturisers, sequestering agents, and natural ingredients.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are preferably selected from polycarboxy acids. The preferred one is ethylene diamine tetraacetic acid, EDTA. Typical useful concentration range for sequestering agents is of 0.01 - 2.5% by weight calculated to the total composition.

The UV filters are that oil and water soluble ones for the purpose of protecting hair and hair colour. In other words, anionic and non-ionic, oily, UV filters are suitably used in the compositions of the present invention. Suitable UV-absorbing substances is are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzaphenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-, dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone-15. The amount of the UV-absorber ranges typically from about 0.01 % to 2.5%, more preferably from 0.05 % to 1 % by weight, calculated to the total composition.

Natural plant extracts are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per so are in particular extracts from leaves, fruits, blossoms, roots, rinds or stems of aloe, pineapple, artichoke, arnica, avocado, valerian, bamboo, henbane, birch, stinging nettle, echinicea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, various "Extrapone®" products, and "Herbasol^{R}". Extracts and the preparation thereof are also described in "Hagers Handbuch der pharmazeutischen Praxis", 4th Ed.

Compositions of the present invention may comprise further at least one compound according to the formula 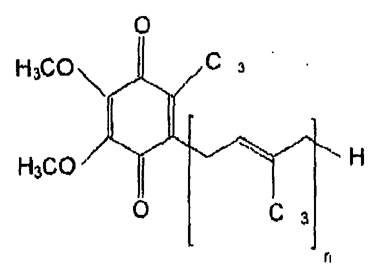 where n is a number from 1 to 10.

The compounds of the above formula are known as Ubiquinone, and also are known as Coenzyme. It should be noted that the compositions of the present invention can certainly comprise more than one ubichinone. Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Aqueous cleansing compositions may further comprise at least one direct dye. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye. Certainly the combination of all three dyestuff categories is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87, and mixtures thereof

Any anionic dye is in principal suitable for the compositions. Suitable examples are such as Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3; Food Yellow No. 8. D&C Brown No. 1, D&C Green No. 5, D&C Green No. 8, D&C Orange No. 4, D&C Orange No. 10, D&C Orange No. 11, D&C Red No. 21, D&C Red No: 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium, and mixtures thereof.

Among those, the preferred anionic dyestuffs are Acid Red 52, Acid Violent 2, Acid Red 33, Acid Orange 4, Acid Red 27 and Acid.Yellow 10 and their salts, and mixtures thereof. The most preferred anionic dyes are Acid Red 52, Acid Violet 2, Acid Red 33, Acid Orange 4 and Acid Yellow 10, and their salts, and mixtures thereof.

Neutral dyes, so called nitro dyes for shading purposes are also optionally contained in the compositions. Suitable ones are HC Blue No.2. HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7. HC Red No.8, HC Red No.9, HC Red No.10, HC Red No.11, HC Red No.13, HC Red No.54, HC, Red No. 14. HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.16,2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid, and mixtures thereof.

Concentration of one or more direct dyes in total is in the range of 0.001 to 5% by weight, preferably 0.01 to 4% more preferably 0.05 to 3% and most preferably 0.1 to 2.5% by weight calculated to total composition. The most preferred among the direct dyes is cationic direct dyes.

It is self-understood that the shampoos according to the invention may comprise other substances customarily used in such compositions such as preservatives, fragrances.

The pH of the compositions according to the present invention is suitable between 2 and 8.0, preferably in the range of 2.5 to 7.0, more preferably 3 to 6.5 and most preferably 4 to 5.5 measured at ambient temperature with a suitable pH meter.

pH of the compositions is adjusted with acidic and alkaline compounds. Acidic compounds can be inorganic and organic acid or their mixtures. Nonlimiting suitable, examples are citric acid, lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Alkaline compounds such as sodium hydroxide can be used to adjust the pH of the compositions.

Aqueous cleansing composition of the present invention preferably comprises one or more thickeners. Suitable ones are ethoxylated polyglyceryl esters with total ethoxy units in the range of 50 to 200 and fatty acyl chain length of 8 to 22 C atoms such as PEG-80 glyceryl cocoate, PEG-90 glyceryl isostearate, PEG-120 glyceryl stearate, PEG-200 glyceryl stearate, PEG-80 glyceryl tallowate, PEG-82 glyceryl tallowate, PEG-130 glyceryl tallowate, and PEG-200 glyceryl tallowate, gylceryl oleate/cocoate and inorganic salt in particular sodium chloride when especially composition comprise alkyl ether sulphate type of surfactants.

Cleansing compositions of the present invention preferably has a viscosity in the range of 500 to 20,000 mPa.s, more preferably 1.000 to 15,000 mPa.s and most preferably 1,500 to 10,000 mPa.s measured at 20°C with a Brookfield viscosimeter using fro example Spindle 5 at appropriate rotation speed.

It is furthermore preferred within the meaning of the present invention that cleansing composition is presented in the form of a kit combined with any other hair cosmetic composition. Thus, another object of the present invention is a kit for hair comprising a hair cosmetic product and a cleansing composition comprising starch derived cationic polymer and at least one sorbitan surfactant.

The following examples are to illustrate the invention, but not to limit, The compositions according to the invention are prepared by mixing the individual components in water, whereby it is also possible to use pre-mixtures of various ingredients.

### Example 1

| | % by weight |
|---|---|
| Sodium lauryl ether sulphate | 14.6 |
| Cocamidopropyl betaine | 3.0 |
| Sodium lauroyl glutamate | 2.5 |
| Decyl glucoside | 2.0 |
| Amylomere H 75 M | 0.5 |
| Sorbitan sesquicaprylate | 2.5 |
| PEG-90 glyceryl isostearate | 3.0 |
| Citric acid/sodium hydroxide | q.s. to pH 5.5 |
| Preservative, fragrance | q.s |
| Water | to 100 |

The above shampoo was judged to have rich and creamy foam in a monadic test by the volunteers. It was furthermore mentioned that it foams very quickly. Additionally, the hair washed was excellently combable, had good shine, volume and body.

Similar results were observed with the compositions below.

### Example 2

| | % by weight |
|---|---|
| Sodium lauryl ether sulphate | 10.6 |
| Cocamidopropyl betaine | 3.0 |
| Sodium lauroyl glutamate | 2.5 |
| Decyl glucoside | 2.0 |
| Sorbitan sesquicaprylate | 2.5 |
| Amylomere H 75 M | 0.5 |
| PEG-90 glyceryl isostearate | 3.0 |
| Citric acid/sodium hydroxide | q.s. to pH 5.5 |
| Preservative, fragrance | q.s |
| Water | to 100 |

### Example 3

| | % by weight |
|---|---|
| Sodium lauryl ether sulphate | 10.6 |
| Cocamidopropyl betaine | 3.0 |
| Sodium lauroyl glutamate | 2.5 |
| Decyl glucoside | 2.0 |
| Amylomere H 75 M | 0.8 |
| Sorbitan sesquicaprylate | 2.5 |
| Glyceryl decyl ether | 0.5 |
| PEG-90 glyceryl isostearate | 3.0 |
| Citric acid/sodium hydroxide | q.s. to pH 5.5 |
| Preservative, fragrance | q.s |
| Water | to 100 |

### Example 4

| | % by weight |
|---|---|
| Sodium lauryl ether sulphate | 10.6 |
| Cocamidopropyl betaine | 3.0 |
| Sodium lauroyl glutamate | 2.5 |
| Decyl glucoside | 2.0 |
| Sorbitan sesquicaprylate | 2.5 |
| Amylomere H 75 M | 0.5 |
| Myristyl alcohol | 0.5 |
| PEG-90 glyceryl isostearate | 3.0 |
| Citric acid/sodium hydroxide | q.s. to pH 5.5 |
| Preservative, fragrance | q.s |
| Water | to 100 |

### Example 5

| | % by weight |
|---|---|
| Sodium lauryl ether sulphate | 10.6 |
| Cocamidopropyl betaine | 3.0 |
| Sodium lauroyl glutamate | 2.5 |
| Decyl glucoside | 2.0 |
| Sorbitan sesquicaprylate | 2.5 |
| Amylomere H 75 M | 0.5 |
| Benzophenone-3 | 0.2 |
| PEG-90 glyceryl isostearate | 3.0 |
| Citric acid/sodium hydroxide | q.s. to pH 5.6 |
| Preservative, fragrance | q.s |

### Example 6

| | % by weight |
|---|---|
| Sodium lauryl ether sulphate | 10.6 |
| Cocamidopropyl betaine | 3.0 |
| Sodium lauroyl glutamate | 2.5 |
| Decyl glucoside | 2.0 |
| Sorbitan sesquicaprylate | 2.5 |
| Amylomere H 75 M | 0.5 |
| Benzophenone-3 | 0.2 |
| PEG-90 glyceryl isostearate | 3.0 |
| Basic red 51 | 0.1 |
| Basic orange 31 | 0.06 |
| Basic yellow 87 | 0.03 |
| Basic red 76 | 0.08 |
| Lactic acid/sodium hydroxide | q.s. to pH 5.5 |
| Preservative, fragrance | q.s |
| Water | to 100 |

The above shampoo gives hair red shine.

## Claims

1. Aqueous cleansing composition for keratin fibres especially for human hair **characterised in that** it comprises starch derived cationic polymer arid at least one sorbitan surfactant.

2. Cleansing composition according to claim 1 **characterised in that** it comprise starch derived cationic polymer at a concentration of 0.1 to 5% by weight, and at least one sorbitan surfactant at a concentration of 0.1 to 15% by weight, all values are calculated to total composition.

3. Composition according to claim 1 **characterised in that** starch derived cationic polymer is an oxidized starch derived cationic polymer.

4. Composition according to claim 1 **characterised in that** starch derived cationic polymer is a hydroxypropyl oxidized starch PG trimonium chloride.

5. Composition according to any of the preceding claims **characterised in that** sorbitan surfactant is selected from sorbitan caprylate, sorbitan cocoate, sorbitan diisostearate, sorbitan dioleate, sorbitan distearate, sorbitan isostearate, sorbitan laurate, sorbitan oleate, sorbitan olivate, sorbitan palmitate, sorbitan palmate, sorbitan sesquicaprylate, sorbitan sesquiisostearate, sorbitan sesquioleate, sorbitan sesquistearate, sorbitan stearate, PEG-20 sorbitan cocoate, PEG-40 sorbitan diisostearate, PEG-2 sorbitan isostearate, PEG-5 sorbitan isostearate, PEG-20 sorbitan isostearate, PEG-40 sorbitan laurate, PEG-10 sorbitan laurate, PEG-44 sorbitan laurate, PEG-75 sorbitan laurate, PEG-80 sorbitan laurate, PEG-3 sorbitan oleate. PEG-6 sorbitan oleate, PEG-20 sorbitan oleate, PEG-40 sorbitan oleate, PEG-80 sorbitan palmitate, PEG-3 sorbitan stearate, PEG-4 sorbitan stearate,
PEG-6 sorbitan stearate, PEG-40 sorbitan stearate, PEG-60 sorbitan stearate, Polysorbate 20, Polysorbate 21, Polysorbate 40, Polysorbate 60, Polysorbate 61, Polysorbate 65. Polysorbate 80, Polysorbate 81, and Polysorbate 85.

6. Cleansing composition according to claim 5 **characterised in that** sorbitan surfactant is sorbitan sesqui ester.

7. Cleansing composition according to claims 5 and 6 **characterised in that** sorbitan surfactant is sorbitan sesquicaprylate.

8. Cleansing composition according to any of the preceding claims **characterised in that** it comprises at least one anionic surfactant preferably at a concentration of 0.5 to 50% by weight calculated to total composition and preferably an ether sulphate type.

9. Cleansing composition according to any of the preceding claims **characterised in that** it comprises additionally at least one non-ionic surfactant, preferably according to the general formula
R₆-O-(R₄O)ₙ-Z_{x,}
wherein R₆ is an alkyl group with 8 to 18 carbon atoms, R is an ethylene or propylene group, Z is a saccharide group with 5 to 6 carbon atoms, n is a number from 0 to 10 and x is a number between 1 and 5.

10. Cleansing composition according to any of the preceding claims **characterised in that** it comprises additionally at least one amphoteric surfactant, preferably selected from betaines, amidoalkyl betaines and sulfobetaines, and their mixtures.

11. Composition according to any of the proceedings claims **characterised in that** it comprises additionally at least one amino acid surfactant preferably selected from the ones according to general structure 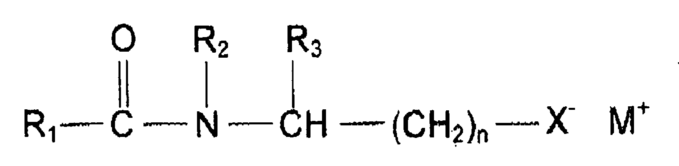 wherein R₁ is a saturated or unsaturated, straight or branched alkyl chain with 7 to 17 C atoms, R₂ is H or a methyl, R₃ is H, COO⁻ M⁺, CH₂COO⁻ M or COOH, n is 0 to 2, X is COO⁻ or SO₃⁻ and M is independent from each other H, sodium or potassium.

12. Composition according to any of the preceding claims **characterised in that** it comprises an additional conditioning agent, preferably selected form cationic polymers, oily substances as conditioning agent selected from silicone oils, either volatile or non-volatile, natural and synthetic oils.

13. Composition according to any of the preceding claims **characterised in that** it comprises at least one UV filter.

14. Composition according to any of the preceding claims **characterised in that** it comprises at least one direct dye.

15. Use of a composition according to any of the preceding claims for cleansing and conditioning hair.
